# EUROPEAN PATENT APPLICATION

(11) **EP 2 186 909 A1**
(43) Date of publication of application: **19.05.2010**
(21) Application number: 09173860.9
(22) Date of filing: 22.10.2009
(51) Int. Cl.: C12Q 1/68

(54) **Highly sensitive multiplex single nucleotide polymorphism and mutation detection using real time ligase chain reaction microarray**

(30) Priority: 23.10.2008 US 256953
(71) Applicant: Honeywell International, Inc., Morristown NJ 07962 (US)
(72) Inventor: Pan, Tao, 201204, Shanghai (CN); Sun, Zhen Hong, 201100, Shanghai (CN); Wang, Wendy, 201100, Shanghai (CN); Liu, Xuanbin, 201702, Shanghai (CN)
(74) Representative: Buckley, Guy Julian

(57) **Abstract**

A method and apparatus for real-time, simultaneous, quantitative measurement of one or more single nucleotide polymorphisms in one or more target nucleic acids is provided. This method involves combining a ligase chain reaction (LCR), a ligase detection reaction (LDR), and/or a polymerase chain reaction (PCR) technique with an evanescent wave technique.

## Description

### BACKGROUND OF THE INVENTION

The most common type of genetic variation is single nucleotide polymorphism (SNP), which may include polymorphism in both DNA and RNA a position at which two or more alternative bases occur at appreciable frequency in the people population(>1%). Base variations with the frequency <1% are called point mutations. For example, two DNA fragments in the same gene of two individuals may contain a difference (e.g., AAGTACCTA to AAGTGCCTA) in a single nucleotide to form a single nucleotide polymorphism (SNP). Typically, there exist many single nucleotide polymorphism (SNP) positions (about 1/1000^{th} chance in whole genome) in a creature's genome. As a result, single nucleotide polymorphism (SNP) and point mutations represent the largest source of diversity in the genome of organisms, for example, a human.

Most single nucleotide polymorphisms (SNP) and point mutations are not responsible for a disease state. Instead, they serve as biological markers for locating a disease on the human genome map because they are usually located near a gene associated with a certain disease. However, many mutations have been directly linked to human disease and genetic disorder including, for example, Factor V Leiden mutations, hereditary haemochromatosis gene mutations, cystic fibrosis mutations, Tay-Sachs disease mutations, and human chemokine receptor mutations. As a result, detection of single nucleotide polymorphisms (SNPs) and similar mutations are of great importance to clinical activities, human health, and control of genetic disease.

Neutral variations are important, for example, because they can provide guideposts in the preparation of detailed maps of the human genome, patient targeted drug prescription, and identify genes responsible for complex disorder. Moreover, since genetic mutation of other species (e.g., bacteria, viruses, etc.) can also be regarded as a type of single nucleotide polymorphism (SNP), the detection of single nucleotide polymorphism (SNP) can also be used to diagnosis the drug resistance, phenotype/genotype, variants and other information of microorganisms that may be useful in clinical, biological, industrial, and other applications.

There are several methods for detecting single nucleotide polymorphism (SNP) and mutations. However, most of the methods are not suitable to be adapted to the platform of automated high-throughput assays or to multiplex screening.

### BRIEF DESCRIPTION OF THE DRAWINGS

Embodiments of the invention may be best understood by referring to the following description and accompanying drawings, which illustrate such embodiments. In the drawings:
FIG. 1 illustrates an exemplary method of using a ligase chain reaction microarray to detect a single nucleotide polymorphism (SNP) in a double-stranded nucleic acid.
FIG. 2 illustrates an exemplary method of using a ligase detection reaction (LDR) optionally coupling with a polymerase chain reaction (PCR) or a reverse transcriptase-polymerase chain reaction (RT-PCR) to detect a single nucleotide polymorphism (SNP) in a double-stranded nucleic acid.

### DETAILED DESCRIPTION OF THE INVENTION

The present invention provides a method and an apparatus for determining the highly sensitive multiplex single nucleotide polymorphism and mutation detection using a real time ligase chain reaction microarray. This method has many advantages including, for example, ease of operation in which all of the steps are integrated on one chip, multiplex single nucleotide polymorphism (SNP) detection in one chip, rapid analysis in less than 3 hours after extracting the DNA, high sensitivity due to amplification and fluorescence detection, labor saving due to automation, and poses very little biosafety hazard because all of reactions are carried out on one disposable chip.

Unless otherwise indicated, the words and phrases presented in this document have their ordinary meanings to one of skill in the art. Such ordinary meanings can be obtained by reference to their use in the art and by reference to general and scientific dictionaries, for example, Webster's Third New International Dictionary, Merriam-Webster Inc., Springfield, MA, 1993 and Hawley's Condensed Chemical Dictionary, 14th edition, Wiley Europe, 2002.

As used herein, the term "about" refers to a variation of 10 percent of the value specified.

As used herein, the term "and/or" refers to any one of the items, any combination of the items, or all of the items with which this term is associated.

As used herein, the singular forms "a," "an," and "the" include plural reference unless the context clearly dictates otherwise.

As used herein, the term "amplicon" refers to the product of a ligase chain reaction (LCR), ligase detection reaction (LDR), or polymerase chain reaction (PCR). Amplicons are pieces of DNA that have been synthesized using amplification techniques (e.g., using a double-stranded DNA and two primers). The amplicon may contain, for example, a primer tagged with a fluorescent molecule at the 5' end as shown in Scheme 1 below.

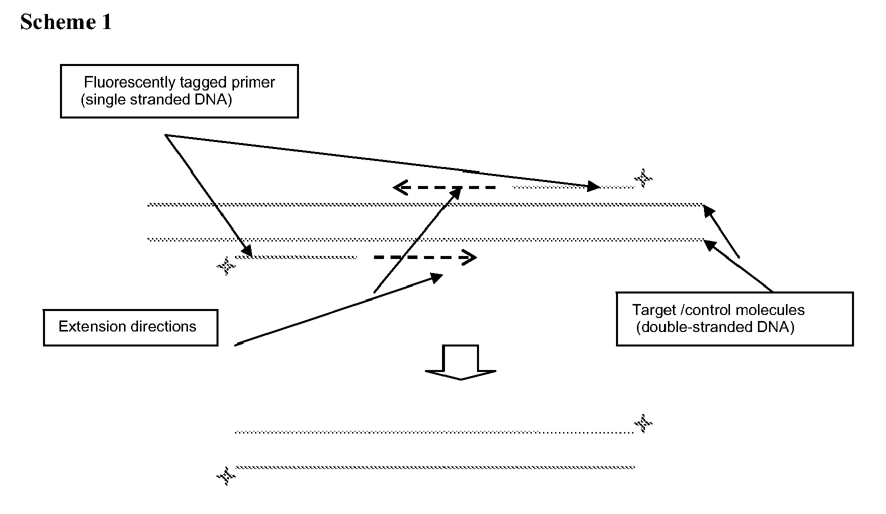
Scheme 1

Fluorescently tagged primer
(single stranded DNA)


Extension directions

Target /control molecules
(double-stranded DNA)


As used herein, the term "buffer solution" refers to a solution that resists changes in the pH. A suitable reaction buffer for a microarray is described in PCT Patent Application Publication No. WO 2008/080254.

As used herein, the term "charge-coupled device" refers to a device for forming images electronically, using a layer of silicon that releases electrons when struck by incoming light.

As used herein, the terms "complementary" or "complementarity" are used in reference to polynucleotides (i.e., a sequence of nucleotides) related by the base-pairing rules. For example, the sequence "A-G-T," is complementary to the sequence "T-C-A." Complementarity may be "partial," in which only some of the nucleic acids' bases are matched according to the base pairing rules. Alternatively, there may be "complete" or "total" complementarity between the nucleic acids. The degree of complementarity between nucleic acid strands has significant effects on the efficiency and strength of hybridization between nucleic acid strands.

As used herein, the term "evanescent" refers to a nearfield standing wave exhibiting exponential decay with distance. As used in optics, evanescent waves are formed when sinusoidal waves are internally reflected off an interface at an angle greater than the critical angle so that total internal reflection occurs. A suitable evanescent wave system that may be used in the practice of this invention is described, for example, in U.S. Patent Application Publication No. 2006/0088844. A suitable microarray reader based on evanescent wave is described in PCT Patent Application Publication No. WO 2008/092291.

As used herein, the term "hybridization" refers to the pairing of complementary nucleic acids. Hybridization and the strength of hybridization (i.e., the strength of the association between the nucleic acids) is impacted by such factors as the degree of complementary between the nucleic acids, stringency of the conditions involved, the melting temperature (Tₘ) of the formed hybrid, and the G:C ratio within the nucleic acids. A single molecule that contains pairing of complementary nucleic acids within its structure is said to be "self-hybridized."

As used herein, the term "ligase" refers to a type of enzyme that can join two nucleotide fragments together when these two nucleotide stands are both complementary and adjacent to a third and long nucleotide strand.

As used herein, the terms "ligase chain reaction (LCR)" and "ligase detection reaction (LDR)" refers to the methods described in M. Wiedmann, et al., "Ligase Chain Reaction (LCR) -Overview and Applications," PCR Methods and Applications, 3. S51-S64, Cold Spring Harbor Laboratory Press, Cold Spring Harbor, NY, USA, 1994.

As used herein, the term "light" refers to an electromagnetic radiation in the wavelength range including infrared, visible, ultraviolet, and X-rays.

As used herein, the term "linker" refers to a carbon chain, which may include other elements that covalently attach two chemical groups together.

As used herein, the term "microarray" is a linear or two-dimensional microarray of discrete regions, each having a defined area, formed on the surface of a solid support. An oligonucleotide probe microarray complementary to the target nucleic acid sequence or subsequence thereof is immobilized on a solid support using one of the display strategies described below. The methods described herein employ oligonucleotide microarrays which comprise target nucleic acid probes exhibiting complementarity to one or more target nucleic acid sequences. Typically, these target nucleic acid probes are DNA and are immobilized in a high-density microarray (i.e., a "DNA chip") on a solid surface.

As used herein, the term "nucleic acid" refers to any nucleic acid containing molecule including, but not limited to, DNA or RNA.

As used herein, the term "nucleic acid sequence" refers to an oligonucleotide, nucleotide or polynucleotide, and fragments or portions thereof, and to DNA or RNA of genomic or synthetic origin which may be single or double stranded, and represent the sense or antisense strand.

As used herein, the terms "nucleoside" and "nucleotide" refer to those moieties which contain not only the known purine and pyrimidine bases, but also other heterocyclic bases that have been modified. Such modifications include, for example, methylated purines or pyrimidines, acylated purines or pyrimidines, alkylated riboses or other heterocycles. In addition, the terms "nucleoside" and "nucleotide" include, for example, those moieties that contain not only conventional ribose and deoxyribose sugars, but other sugars as well. Modified nucleosides or nucleotides also include, for example, modifications on the sugar moiety, e.g., wherein one or more of the hydroxyl groups are replaced with halogen atoms or aliphatic groups, or are functionalized as ethers, amines, or the like.

As used herein, the term "optical detection path" refers to a configuration or arrangement of detection means to form a path whereby electromagnetic radiation is able to travel from an external source to a means for receiving radiation, wherein the radiation traverses the reaction chamber.

As used herein, the term "polymerase chain reaction (PCR)" refers to the method of K. B. Mullis, U.S. Patent Nos. 4,683,195, 4,683,202, and 4,965,188. This process for amplifying the target sequence consists of introducing a large excess of two oligonucleotide primers to the DNA mixture containing the desired target sequence, followed by a precise sequence of thermal cycling in the presence of a DNA polymerase. The two primers are complementary to their respective strands of the double-stranded target sequence. To effect amplification, the mixture is denatured and the primers annealed to their complementary sequences within the target molecule. Following annealing, the primers are extended with a polymerase so as to form a new pair of complementary strands. The steps of denaturation, primer annealing, and polymerase extension can be repeated many times (i.e., denaturation, annealing, and extension constitute one "cycle" and there can be numerous "cycles") to obtain a high concentration of an amplified segment of the desired target sequence. The length of the amplified segment of the desired target sequence is determined by the relative positions of the primers with respect to each other, and therefore, this length is a controllable parameter. By virtue of the repeating aspect of the process, the method is referred to as the "polymerase chain reaction" (hereinafter "PCR"). Because the desired amplified segments of the target sequence become the predominant sequences (in terms of concentration) in the mixture, they are said to be "PCR amplified."

With PCR, it is possible to amplify a single copy of a specific target sequence in genomic DNA to a level detectable by several different methodologies (e.g., hybridization with a labeled probe; incorporation of biotinylated primers followed by avidin-enzyme conjugate detection; incorporation of ³²P-labeled deoxynucleotide triphosphates, such as dCTP or dATP, into the amplified segment). In addition to genomic DNA, any oligonucleotide or polynucleotide sequence can be amplified with the appropriate set of primer molecules. In particular, the amplified segments created by the PCR process are, themselves, efficient templates for subsequent PCR amplifications.

As used herein, the term "predetermined wavelength of light" refers to light of a particular wavelength emitted by a label that indicates the presence of the label. A particular wavelength of light may contain a range of wavelengths (e.g.,+/-5 nm or more) that contains the wavelength at which emission of the label is at a maximum.

As used herein, the term "primer" refers to a single-stranded polynucleotide capable of acting as a point of initiation of template-directed DNA synthesis under appropriate conditions.

As used herein, the term "probe" refers to a nucleic acid capable of binding to a target nucleic acid of complementary sequence through one or more types of chemical bonds, usually through complementary base pairing, usually through hydrogen bond formation, thus forming a duplex structure. A probe binds or hybridizes to a "probe binding site." A probe can be labeled with a detectable label to permit facile detection of the probe, particularly once the probe has hybridized to its complementary target. A label attached to the probe can include any of a variety of different labels known in the art that can be detected by, for example, chemical or physical means. Labels that can be attached to probes may include, for example, fluorescent and luminescence materials. Probes can vary significantly in size. Some probes are relatively short. Generally, probes are, for example, at least 7 to 15 nucleotides in length. Other probes are, for example, at least 20, 30 or 40 nucleotides long. Still other probes are somewhat longer, being at least, for example, 50, 60, 70, 80, 90 nucleotides long. Yet other probes are longer still, and are at least, for example, 100, 150, 200 or more nucleotides long. Probes can be of any specific length that falls within the foregoing ranges as well.

As used herein, the term "reactor" refers to a device, which can be used in any number of chemical processes involving a fluid.

As used herein, the term "reverse transcriptase-polymerase chain reaction (RT-PCR)" refers to the combination of two reactions: 1) generate a complementary DNA fragment using RNA as template with the help of reverse transcriptase or similar enzymes, and 2) polymerase chain reaction (PCR) amplification of DNA generated in the previous step. Thus, RNA can be converted to DNA and subsequently amplified.

As used herein, the term "sequence variation" refers to differences in nucleic acid sequence between two nucleic acids. For example, a wild-type structural gene and a mutant form of this wild-type structural gene may vary in sequence by the presence of single base substitutions and/or deletions or insertions of one or more nucleotides. These two forms of the structural gene are said to vary in sequence from one another. A second mutant form of the structural gene may exist. This second mutant form is said to vary in sequence from both the wild-type gene and the first mutant form of the gene.

As used herein, the term "single nucleotide polymorphism (SNP)" refers to a DNA sequence variation occurring when a single nucleotide - A, T, C, or G - in the genome (or other shared sequence) differs between members of a species (or between paired chromosomes in an individual).

As used herein, the term "target nucleic acid" refers to a polynucleotide, which includes, for example, at least two nucleotides. The polynucleotide is genetic material including, for example, DNA/RNA, mitochondrial DNA, rRNA, tRNA, mRNA, viral RNA, bacterial DNA or RNA, plasmid DNA, and eukaryote or prokaryote DNA or RNA.

As used herein, the term "Tₘ" refers to the "melting temperature." The melting temperature is the temperature at which a population of double-stranded nucleic acid molecules becomes half dissociated into single strands. The equation for calculating the Tₘ of nucleic acids is well known in the art. As indicated by standard references, a simple estimate of the Tₘ value may be calculated by the equation: Tₘ = 81.5 +0.41 (% G+C), when a nucleic acid is in aqueous solution at 1 M NaCl (*see,* e.g., Anderson and Young, Quantitative Filter Hybridization, in Nucleic Acid Hybridization (1985)). Other referencesmay include, for example, more sophisticated computations that take structural as well as sequence characteristics into account for the calculation of Tₘ. As used herein, the melting temperature (Tₘ) represents the temperature at which the hybridization signal will be reduced to 50% of the saturated hybridization signal.

As used herein, the term "thermostable" refers to an enzyme, such as a 5' nuclease, indicates that the enzyme is functional or active (i.e., can perform catalysis) at an elevated temperature, for example, at about 55 °C or higher.

The present invention provides a quantitative method for determining one or more single nucleotide polymorphisms in one or more double-stranded target nucleic acids. The method includes: (a) independently annealing one or more primer sets to one or more pairs of complementary single-stranded target nucleic acids, wherein each primer set comprises: a tag primer comprising a recognition tag; a reporter primer comprising a fluorescent tag; wherein each tag primer and each reporter primer have nucleic acid sequences complementary to a first single-strand of the one or more pairs of complementary single-stranded target nucleic acids, and wherein each tag primer and each reporter primer anneal in tandem on the first single-strand; (b) independently ligating each tag primer to each reporter primer annealed to the complementary single-stranded sequences in the target nucleic acids to provide one or more fluorescently tagged target amplicons;(c) independently hybridizing the one or more fluorescently tagged target amplicons to one or more anti-recognition tag primer probes in independent areas on an upper surface of a substrate; (d) independently activating one or more fluorescence responses from the one or more fluorescently tagged target amplicons hybridized to the one or more anti-recognition tag primer probes in independent areas on the upper surface of the substrate using an evanescent wave of a predetermined wavelength; and (e) independently detecting the one or more fluorescence responses to quantitatively determine single nucleotide polymorphisms in the one or more double-stranded target nucleic acids.

In one embodiment, the method further includes amplifying the one or more double-stranded target nucleic acids by a polymerase chain reaction (PCR) or a reverse transcriptase-polymerase chain reaction (RT-PCR), wherein each of the one or more double-stranded target nucleic acids has at least two different nucleotides at one single nucleotide polymorphism (SNP) site. In another embodiment, the method further includes denaturing the one or more double-stranded target nucleic acids to provide one or more pairs of complementary single-stranded target nucleic acids. In yet another embodiment, the method further includes analyzing the one or more fluorescence responses.

In one embodiment, each primer set further includes a first reverse primer and a second reverse primer, wherein the first reverse primer and the second reverse primer have nucleic acid sequences complementary to the second single-strand of the one or more pairs of complementary single-stranded target nucleic acids, and wherein the first reverse primer and a second reverse primer anneal in tandem on the second single-strand.

In another embodiment, the method further includes ligating each first reverse primer to each second reverse primer to provide one or more untagged target amplicons. In one embodiment, the annealing occurs during a ligase chain reaction or a ligase detection reaction.

In one embodiment, the one or more anti-recognition tag primer probes each comprise a DNA sequence, a RNA sequence, a protein, or a combination thereof. In another embodiment, the recognition tag includes a polynucleotide, a protein, a metal ion, or a combination thereof. In yet another embodiment, the fluorescent tag includes a quantum dot, an enzyme, a nanoparticle, a dye, a pigment, or a combination thereof.

In one embodiment, the substrate includes silicon, glass, quartz, a ceramic, a rubber, a metal, a polymer, a hybridization membrane, or a combination thereof. In another embodiment, the substrate is chemically modified with a reagent selected from a silane, avidin, poly-L-lysine, streptavidin, a polysaccharide, a mercaptan, or a combination thereof. In yet another embodiment, the one or more anti-recognition tag primer probes are printed and immobilized onto the substrate using a micro-array printer.

In one embodiment, the one or more anti-recognition tag primer probes each comprise a linker with a sulfhydryl (RSH), amino (NH₂), hydroxyl (OH), carboxaldehyde (CHO), or carboxylic acid (COOH) group at the 3' end. In another embodiment, the linker includes about a ten nucleotide random oligomer. In yet another embodiment, the one or more anti-recognition tag primer probes are immobilized onto a silanized glass substrate with the sulfhydryl (RSH) group at the 3' end.

The present invention provides an apparatus. The apparatus includes: a closed reactor including: a substrate having first and second planar opposing surfaces, the substrate having a cavity and a refractive index greater than a refractive index of water; a buffer layer arranged over the first planar surface of the substrate; a cover plate arranged over the buffer layer and the cavity, the cover plate in combination with the cavity and the buffer layer defining a reaction chamber; and at least one inlet port and at least one outlet port to communicate with the reaction chamber through the substrate to enable the passage of fluid from an external source into and through the reaction chamber; a temperature control system coupled to the closed reactor to control the temperature of a buffer solution contained within the closed reactor, wherein the buffer solution is substantially in contact with the first surface of the substrate and being capable of sustaining a plurality of ligation reactions, a plurality of hybridization reactions, and containing one or more primer sets and one or more double-stranded target nucleic acids; a light source coupled to the closed reactor to provide a ray of light having a wavelength chosen to activate one or more fluorescently tagged target amplicons hybridized to one or more anti-recognition tag primer probes immobilized in independent areas on the first surface of the substrate, incident on an interface between the substrate and the buffer solution at an angle chosen to propagate an evanescent wave into the buffer solution; and a detector coupled to the closed reactor to detect the one or more fluorescent responses emitted by one of the one or more fluorescently tagged target amplicons hybridized to one or more anti-recognition tag primer probes immobilized in independent areas on the first surface of the substrate.

In one embodiment, the detector is mobile and capable of sequentially detecting fluorescent light emitted by the one or more fluorescently tagged target amplicons attached to the one or more anti-recognition tag primer probes. In another embodiment, the closed reactor is mobile and capable of being sequentially addressed by the detector. In yet another embodiment, the detector includes a camera, a charge-coupled device, a charge-injection device, a complementary metal-oxide-semiconductor (CMOS) device, a video camera, a silicon photo-cell, a photodiode, an avalanche photodiode, a photo-multiplier tube, or a combination thereof.

In one embodiment, the method utilizes the high sensitivity of the ligase chain reaction (LCR)/ ligase detection reaction (LDR) to selectively amplify the matched single nucleotide polymorphism (SNP) fragments in a sample and to add a fluorescence signal to the amplified fragments. Since the ligase chain reaction (LCR) is highly sensitive, only matched fragment can be amplified.

In one embodiment, the method also utilizes a real-time ligase chain reaction (LCR) microarray based on amplification and hybridization of multiple target nucleotide acid sets in a microarray chamber. Because amplified single nucleotide polymorphism (SNP) fragments may contain a code-like tag that can hybridize to surface immobilized anti tag probes, the existence of different single nucleotide polymorphism (SNP) variants may be easily detected by examining the specific probe areas.

In one embodiment, the method also utilizes real-time detection by evanescent wave, which can detect the hybridization signal during hybridization process and eliminate the washing steps. This method may be easily expanded for multiplex single nucleotide polymorphism (SNP) detection. Typically, for each single nucleotide polymorphism (SNP) site, a set of tag primers, report primer, reverse primer 1, reverse primer 2, anti tag probes are prepared and the anti tag probes are immobilized on one microchip chip. For the ligase detection reaction (LDR) mode, for each single nucleotide polymorphism (SNP) site, a set of tag primers, report primer, anti tag probes are prepared and the anti tag probes are immobilized on one microchip chip.

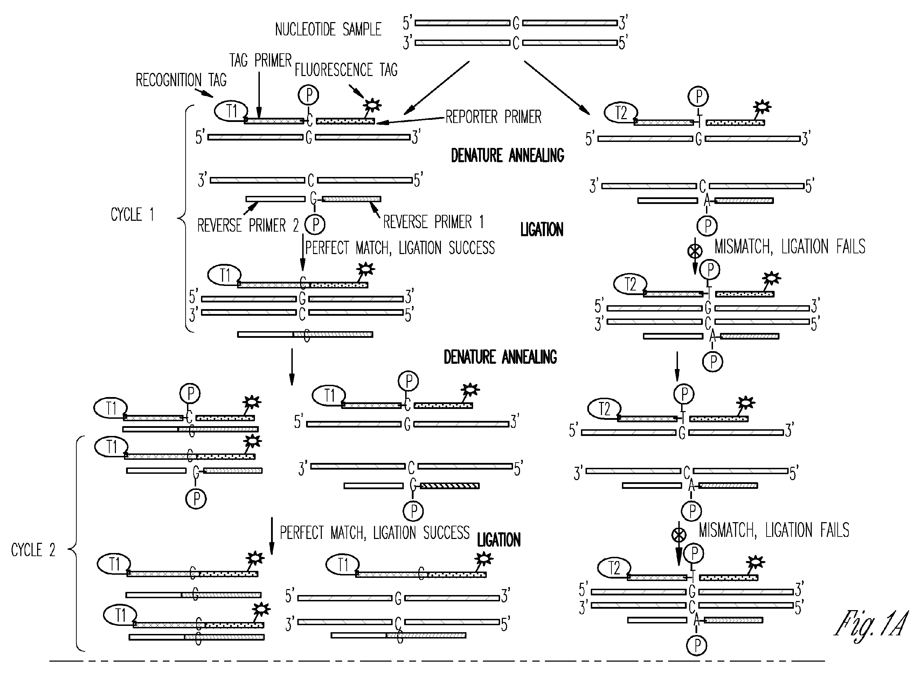

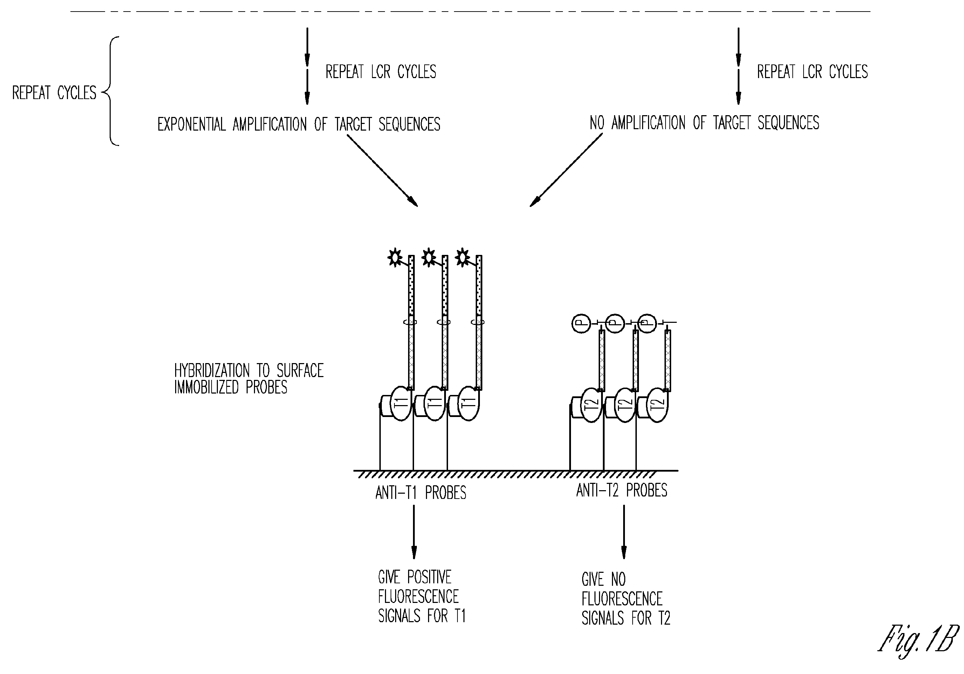

An example of a ligase chain reaction (LCR) microarray for the detection of a single nucleotide polymorphism (SNP) using an exponential amplification of target sequences is illustrated in FIG. 1. For example, two strands of *E.aerogenes* are obtained from a pure culture of clinical samples, which have already been identified using conventional biochemical methodologies. The 16S rDNA of these two strands of *E. aerogenes* are purified and the sequencing results are as followed:

Strand A
5'CaaGCCTGATGCAGCCATGCCGCGTGTATGAAGAAGGCCTTCGGGTT
GTAAAGTACTTTCAGCGAGGAGGAAGGCGTTAAGGTTAATAACCTTG
GCGATTGACGTTACTCGCAGAAGAAGCACCGGCTAACTCCGTGCCAG
CAGCCGCGGTAATA 3'

Strand B
5'caAGCCTGATGCAGCCATGCCGCGTGTATGAAGAAGGCCTTCGGGTT
GTAAAGTACTTTCAGCGAGGAGGAAGGCATTAAGGTTAATAACCTTG
GCGATTGACGTTACTCGCAGAAGAAGCACCGGCTAACTCCGTGCCAG
CAGCCGCGGTAATA 3'

Within the sequencing area, only one single base difference exists: The base guanine (G) in strand A is replaced by base adenine (A) in strand B (as indicated by the underlining above). To identify this difference, four types of primers are designed and fabricated using conventional techniques:
Tag Primer 1
   Tag 1-5'-AAGTACTTTCAGCGAGGAGGAAGGC**G**-3'
Tag Primer 2
   Tag 2-5'-AAGTACTTTCAGCGAGGAGGAAGGC**A**-3'

If the recognition tag 1 and recognition tag 2 are used to discriminate Tag Primer 1 from Tag Primer 2, then the recognition tags (e.g., recognition tag 1 and recognition tag 2) may be, for example, a polynucleotide, a protein or a peptide, an antigen, a metal ion, or a combination thereof. After the ligase chain reaction (LCR) cycle is completed, recognition tag 1 and recognition tag 2 will be attracted to surface modified anti-recognition tag 1 and anti-recognition tag 2 probes for detection. If the recognition tag 1 and recognition tag 2 are nucleotides fragments, the anti-recognition tag probes (e.g., anti-recognition tag probe 1 and anti-recognition tag probe 2) should contain nucleotide fragments that are complementary to corresponding recognition tags (i.e., anti-recognition tag 1 probe to recognition tag 1; anti-recognition tag 2 probe to recognition tag 2).
Report Primer: B
   Fluorescence tag-5'-TTAAGGTTAATAACCTTGGCGATTGACGTTAC-3'
Reverse Primer 1-1
   5'-GTAACGTCAATCGCCAAGGTTATTAACCTTAA**C**-3'
Reverse Primer 1-2
   5'-GTAACGTCAATCGCCAAGGTTATTAACCTTAA**T**-3'
Reverse Primer 2
   5'-GCCTTCCTCCTCGCTGAAAGTACTT-3'

Typically, Reporter Primer B is labeled with a fluorescence tag, for example, a quantum dot, an enzyme (e.g., horseradish peroxidase), nanoparticles (e.g., Au), a dye (e.g., cyanine 5 (Cy5), cyanine 3 (Cy3), fluorescein isothiocyanate (FITC)), a pigment, or a combination thereof.

These probes and tags may be designed using software such as Array Designer 4 (Premier Biosoft International, Palo Alto, CA, USA), which can screen probes for their sequence features, thermodynamic properties and secondary structures. In one embodiment, the length of probes and tags should be about 20-35 mer, wherein one mer equals one nucleotide base. In another embodiment, the melting temperature (Tₘ) of hybridizations of probe to tag should be similar to or higher than the one needed for ligase chain reaction (LCR).

In one embodiment, anti-recognition tag probes (if DNA or RNA) may be synthesized with an amino (e.g., -NH₂) group at 3' end. To reduce potential space hindrance, a linker made of 10 nucleotide random oligomer may be added at 5' end. Correspondingly, a -NH₂ group may be modified at 3' end of the linker. The folding conformations of the probes with linkers can be calculated, for example, by the online computation server Mfold (*see*, e.g., http://mfold.bioinfo.rpi.edu/cgi-bin/rna-forml.cgi). The results with high ΔG are not used.

The anti-recognition tag probes are immobilized onto the modified silane glass with NH₂-group, using an aspirate-dispensing arrayer, for example, a Biodot Arrayer (Cartesian Technologies, Irvine, CA, USA) or similar contact-spotting arrayers. The anti-recognition tag probes can be arranged in the format of an array on the surface of the silane glass.

The microarray chip (e.g., glass, plastic, etc.) with immobilized anti-recognition tag probes array can be assembled with a plastic piece to form a closed reaction chamber, inside which the ligase chain reaction (LCR) reaction, hybridization reaction, and detection may be carried out simultaneously. A DNA sample extracted from real sample (e.g., a clinical sample, an environmental sample, a food or drink sample, an industrial sample, etc.) is added to reaction chamber, together with ligase chain reaction (LCR) buffer to form a ligase chain reaction (LCR) mixture. A typical ligase chain reaction (LCR) mixture contains 20 mM Tris-HCl (pH 8.3), 25 mM KCl, 10mM MgCl₂, 0.5 mM NAD+ (nicotinic adenine dinucleotide, a cofactor for ligase enzyme), 0.01% Triton X-100, the discriminating primers (25 nM each), fluorescently labeled primer (25 nM each), mixture of extracted DNA sample, and 2 U/L of a ligase enzyme, for example, a thermostable ligase enzyme. The chamber may be sealed with a set of rubber plugs before the amplification reaction. The chamber is heated and cooled with a heating/cooling apparatus (e.g., semi-conductor cooler, heat tube, cooling fan, etc.) to temperatures, which are routinely used in ligase chain reaction (LCR). For example, in a typical ligase chain reaction (LCR), the ligase chain reaction (LCR) mixture is preheated to 94 °C for 2 minutes and subsequently subjected to 30 or more ligase chain reaction (LCR) thermal cycles using the following temperatures cycles: 94 °C for 30 seconds; 60°C for 2 minutes. At the end of each ligase chain reaction (LCR) cycle, the temperature may be adjusted to insure that the tag primer hybridizes to surface modified anti-recognition tag probes for detection. During each cycle of ligase chain reaction (LCR), only matched primers may be ligated. For example, if a DNA sample only contains guanine (G) at the single nucleotide polymorphism (SNP) site, Tag primer 1 may be ligated to report primer 1; and reverse primer 1-I can be ligated to reverse primer 2. Tag primer 2 may not be ligated to report primer 1 because the last nucleotide adenosine (A) does not match the guanosine (G) in the sample. For the same reason, reverse primer 1-2 can not be ligated to reverse primer 2. As a result, after first ligase chain reaction (LCR) cycle, only guanine (G) containing single nucleotide polymorphism (SNP) subtype is amplified. If more ligase chain reactions (LCRs) are performed, it may be found that the amplification of guanine (G) containing the single nucleotide polymorphism (SNP) subtype is amplified exponentially, while the adenine (A) containing single nucleotide polymorphism (SNP) subtype is not amplified. Further, if the tag primer is ligated to the reporter primer, a fluorescence response may be produced upon excitation.

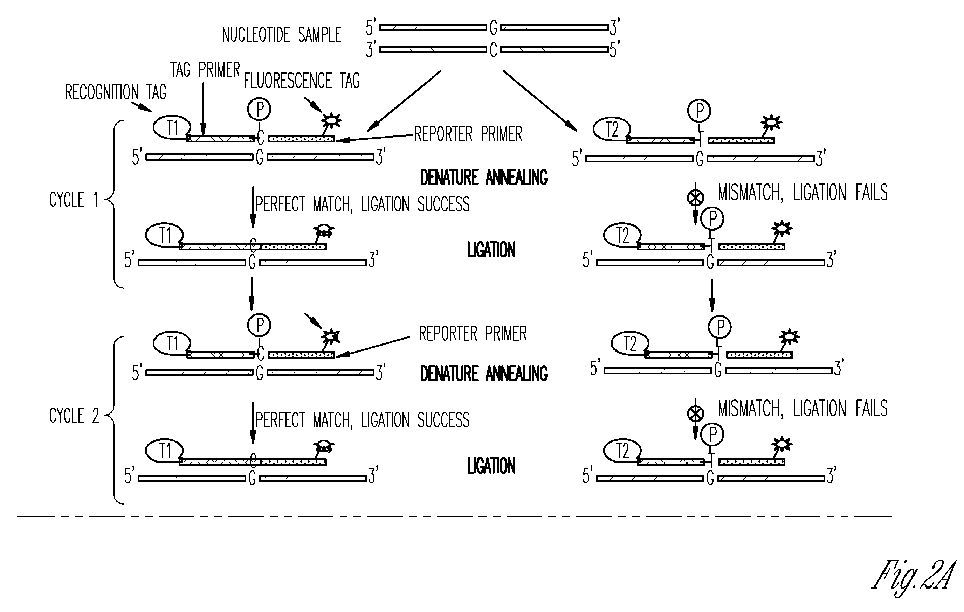

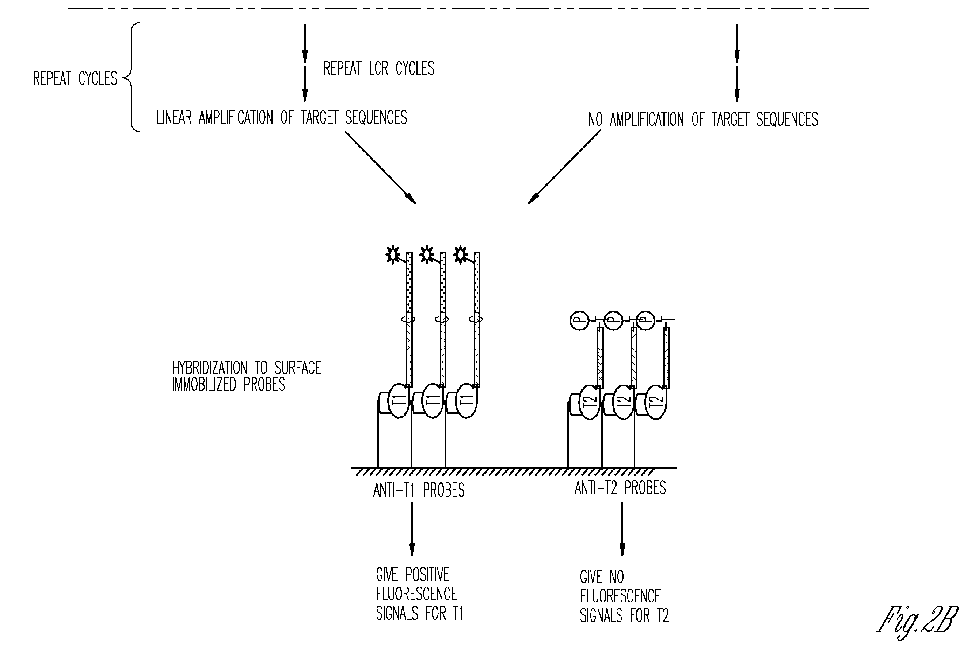

After each ligase chain reaction (LCR) cycle, a hybridization step is performed to react recognition tags with specific anti-recognition tag probes immobilized in the glass substrate, where the primer with Tag 1 will hybridize to anti-recognition Tag 1 probe, and the primer with Tag 2 will hybridize to anti-recognition Tag 2 probe. In this case, Tag 1 primer may provide a fluorescence response because it is ligated to a report primer. The hybridization between fluorescent-labeled amplicons and probes may be detected by an evanescent wave during the end of each ligase chain reaction (LCR) cycle. This will allow for real time information after each ligase chain reaction (LCR) cycle. An example of a ligase chain reaction (LCR) microarray for the detection of a single nucleotide polymorphism (SNP) using a linear amplification of target sequences is illustrated in FIG. 2. The operation of a ligase detection reaction (LDR) based single nucleotide polymorphism (SNP) detection is similar to the ligase chain reaction (LCR) based single nucleotide polymorphism (SNP) detection described above, except that no reverse primer is required. In some cases, a polymerase chain reaction (PCR) amplification of target DNA sequences is performed before the ligase chain reaction (LCR)/ ligase detection reaction (LDR). This polymerase chain reaction (PCR) may be carried out in the same chamber with ligase chain reaction (LCR)/ ligase detection reaction (LDR), or may be performed separately. Although the above discussion is directed toward on single nucleotide polymorphism (SNP), one of skill in the art may easily recognize that this technique may be extend to detect multiplex single nucleotide polymorphisms (SNPs) in parallel in one reaction by using several different sets of anti-recognition tag probes and primers.

All patents and publications referenced or mentioned herein are indicative of the levels of skill of those skilled in the art to which the invention pertains, and each such referenced patent or publication is hereby incorporated by reference to the same extent as if it had been incorporated by reference in its entirety individually or set forth herein in its entirety. Applicants reserve the right to physically incorporate into this specification any and all materials and information from any such cited patents or publications.

## Claims

1. A quantitative method for determining one or more single nucleotide polymorphisms in one or more double-stranded target nucleic acids comprising:
(a) independently annealing one or more primer sets to one or more pairs of complementary single-stranded target nucleic acids,
wherein each primer set comprises:
a tag primer comprising a recognition tag;
a reporter primer comprising a fluorescent tag;
wherein each tag primer and each reporter primer have nucleic acid sequences complementary to a first single-strand of the one or more pairs of complementary single-stranded target nucleic acids, and
wherein each tag primer and each reporter primer anneal in tandem on the first single-strand;
(b) independently ligating each tag primer to each reporter primer annealed to the complementary single-stranded sequences in the target nucleic acids to provide one or more fluorescently tagged target amplicons;
(c) independently hybridizing the one or more fluorescently tagged target amplicons to one or more anti-recognition tag primer probes in independent areas on an upper surface of a substrate;
(d) independently activating one or more fluorescence responses from the one or more fluorescently tagged target amplicons hybridized to the one or more anti-recognition tag primer probes in independent areas on the upper surface of the substrate using an evanescent wave of a predetermined wavelength; and
(e) independently detecting the one or more fluorescence responses to quantitatively determine single nucleotide polymorphisms in the one or more double-stranded target nucleic acids.

2. The quantitative method of claim 1, further comprising amplifying the one or more double-stranded target nucleic acids by a polymerase chain reaction (PCR) or a reverse transcriptase-polymerase chain reaction (RT-PCR), wherein each of the one or more double-stranded target nucleic acids has at least two different nucleotides at one single nucleotide polymorphism (SNP) site.

3. The quantitative method of claim 1, further comprising denaturing the one or more double-stranded target nucleic acids to provide one or more pairs of complementary single-stranded target nucleic acids.

4. The quantitative method of claim 1, further comprising analyzing the one or more fluorescence responses.

5. The quantitative method of claim 1, wherein each primer set further comprises a first reverse primer and a second reverse primer,
wherein the first reverse primer and the second reverse primer have nucleic acid sequences complementary to the second single-strand of the one or more pairs of complementary single-stranded target nucleic acids, and
wherein the first reverse primer and a second reverse primer anneal in tandem on the second single-strand.

6. The quantitative method of claim 5, further comprising ligating each first reverse primer to each second reverse primer to provide one or more untagged target amplicons.

7. The quantitative method of claim 1, wherein the annealing occurs during a ligase chain reaction or a ligase detection reaction.

8. The quantitative method of claim 1, wherein the one or more anti-recognition tag primer probes each comprise a DNA sequence, a RNA sequence, a protein, or a combination thereof.

9. The quantitative method of claim 1, wherein the recognition tag comprises a polynucleotide, a protein, a metal ion, or a combination thereof, or wherein the fluorescent tag comprises a quantum dot, an enzyme, a nanoparticle, a dye, a pigment, or a combination thereof.

10. An apparatus comprising:
a closed reactor comprising:
a substrate having first and second planar opposing surfaces, the substrate having a cavity and a refractive index greater than a refractive index of water;
a buffer layer arranged over the first planar surface of the substrate;
a cover plate arranged over the buffer layer and the cavity, the cover plate in combination with the cavity and the buffer layer defining a reaction chamber; and
at least one inlet port and at least one outlet port to communicate with the reaction chamber through the substrate to enable the passage of fluid from an external source into and through the reaction chamber;
a temperature control system coupled to the closed reactor to control the temperature of a buffer solution contained within the closed reactor, wherein the buffer solution is substantially in contact with the first surface of the substrate and being capable of sustaining a plurality of ligation reactions, a plurality of hybridization reactions, and containing one or more primer sets and one or more double-stranded target nucleic acids;
a light source coupled to the closed reactor to provide a ray of light having a wavelength chosen to activate one or more fluorescently tagged target amplicons hybridized to one or more anti-recognition tag primer probes immobilized in independent areas on the first surface of the substrate, incident on an interface between the substrate and the buffer solution at an angle chosen to propagate an evanescent wave into the buffer solution; and
a detector coupled to the closed reactor to detect the one or more fluorescent responses emitted by one of the one or more fluorescently tagged target amplicons hybridized to one or more anti-recognition tag primer probes immobilized in independent areas on the first surface of the substrate.
